# EUROPEAN PATENT SPECIFICATION

(11) **EP 1 987 786 B1**
(45) Date of publication and mention of the grant of the patent: **06.11.2013**
(21) Application number: 08251594.1
(22) Date of filing: 01.05.2008
(51) Int. Cl.: A61B 17/16

(54) **Flip retrograde cutting instrument**
Ausklappbares rückseitiges Schneidinstrument
Instrument basculant de découpe rétrograde

(30) Priority: 02.05.2007 US 915607 P
(43) Date of publication of application: 05.11.2008
(73) Proprietor: Arthrex, Inc., Naples, FL 34108-1945 (US)
(72) Inventor: Sterrett, Jerry, Naples FL 34119-8653 (US); Koogle, David, Naples FL, 34110-2835 (US); Iannarone, Ronald C., Aiken South Carolina, 29803-1626 (US); Albertorio, Ricardo, Naples FL, 34110 (US)
(74) Representative: Robertson, James Alexander

(56) References cited:
- EP-A- 0 241 240
- EP-A- 1 785 103
- FR-A- 2 613 212
- US-A- 5 429 504
- US-A- 5 928 239
- US-A- 5 941 706
- US-A1- 2001 034 526
- US-A1- 2005 261 684
- US-B1- 6 884 246

## Description

### BACKGROUND OF THE INVENTION

### 1. Field of the Invention:

The present invention relates to arthroscopic surgical instruments and, more specifically, to a flip retrograde cutting instrument for retrograde repairs and reconstructions.

### 2. Description of the Related Art:

During arthroscopic surgery, a small incision is made in the skin covering the arthroscopic site or joint, and a cannula is inserted in the incision to provide a pathway for surgical instruments to be placed in the joint and manipulated through arthroscopic visualization. Surgical instruments inserted through cannulas must be long and thin - this presents limitations on instruments for cutting tissue, as the diameter of the cannula ordinarily limits the width of the cutting implement. Such an instrument is disclosed in US 2001/0034526 A1 or in US 2005/0 261 684 A1, the contents of which form the basis for the preamble of claim 1 appended hereto.

Retrograde drilling of sockets and tunnels for ACL reconstruction is known and described, for example, in U.S. Patent Application Publication No. 2007/0233138, entitled "Method and Apparatus for ACL Reconstruction using Retrograde Cutter." In such a method, sockets in bone created by retrograde cutting. A rotary cutter, mounted onto an insertion post of a guide, is inserted through an anteromedial portal into the knee joint. A drill pin is drilled through the tibia and advanced until it contacts and engages a cannulation in the rotary cutter on the guide. Further rotation of the drill pin disengages the rotary cutter from the guide. The retrograde drill pin is then retracted and simultaneously rotated for retrograde cutting of a socket or tunnel of desired depth in the tibia. A similar method can be used for drilling a femoral socket or tunnel. A need exists for a surgical cutting instrument that can be used arthroscopically for retrograde drilling of tunnels or sockets in bone without requiring a rotary cutter and drill pin. As with all arthroscopic instruments, the surgical cutting instrument must be configured for insertion through a narrow cannula, but able to cut a relatively wide tunnel or socket.

A need also exists for a surgical cutter that is stable during knee arthroscopy and that provides drilling of femoral and tibial sockets or tunnels independently of one another and minimizes incisions of distal cortices and reduces intraarticular bone fragmentation of tunnel rims.

### SUMMARY OF THE INVENTION

The present invention provides a flip retrograde cutter as defined in claim 1 having a blade, preferably a flip blade, that is configured to articulate between at least a first "straight" position substantially parallel to a longitudinal axis of the flip retrograde cutter, and at least a second "flip" position about 90° relative to the longitudinal axis of the flip retrograde cutter.

The present invention provides a flip retrograde cutter that creates a recipient site socket from the inside out, i.e., using a retrograde technique, with minimal incisions of distal cortices and reduced intraarticular bone fragmentation of tunnel rims.

The flip retrograde cutter of the present invention may be employed in a retrograde manner, to form a recipient socket (to accommodate an osteochondral transplant, or to allow retrograde fixation of a graft within two sockets, for example). Formation of the recipient socket begins by inserting the flip retrograde cutter in the "straight" configuration into the joint space, preferably from the outside in, through a small diameter tunnel. A locking tube of the instrument is then retracted so that the blade can be articulated into the "flip" configuration, i.e., into a position other than the "straight" position at about 90 degrees to the longitudinal axis of the instrument. The device is locked in the "flip" position by tightening the locking tube. A socket is created by conducting a drilling operation, i.e., by rotating the instrument, while the device is pulled outwardly.

Other features and advantages of the invention will become apparent from the following description of the invention, which refers to the accompanying drawings.

### BRIEF DESCRIPTION OF THE DRAWINGS

Figures 1A and 1B illustrate perspective views of a flip retrograde cutter of the present invention showing the blade in straight and flip positions.

Figures 2A-2B illustrate an exploded view and a perspective view of the flip retrograde cutter of the present invention.

Figures 3A-3C illustrate various views of a driver end of the flip retrograde cutter of the present invention.

Figure 4 illustrates a perspective view of a nut of the flip retrograde cutter of the present invention.

Figure 5 illustrates a perspective view of a hub of the flip retrograde cutter of the present invention.

Figures 6A-6B illustrate various views of a locking tube of the flip retrograde cutter of the present invention.

Figure 7 illustrates a perspective view of a blade/cutter tip of the flip retrograde cutter of the present invention.

Figures 8A-8B illustrate various views of a shaft of the flip retrograde cutter of the present invention.

### DETAILED DESCRIPTION OF THE PREFERRED EMBODIMENT

In the following detailed description, reference is made to various specific embodiments in which the invention may be practiced. These embodiments are described with sufficient detail to enable those skilled in the art to practice the invention, and it is to be understood that other embodiments may be employed, and that structural and logical changes may be made without departing from the scope of the present invention.

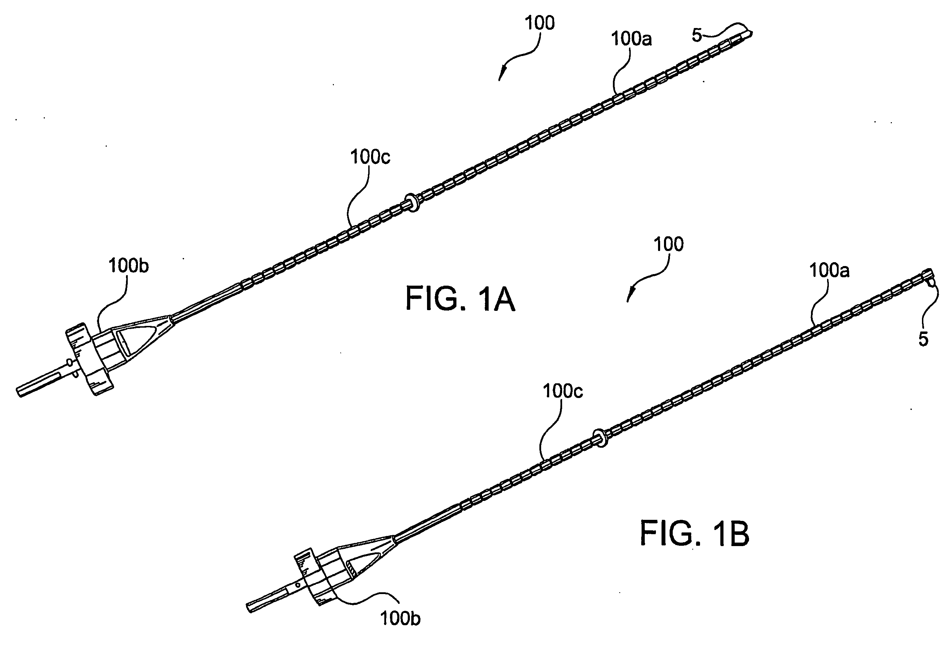

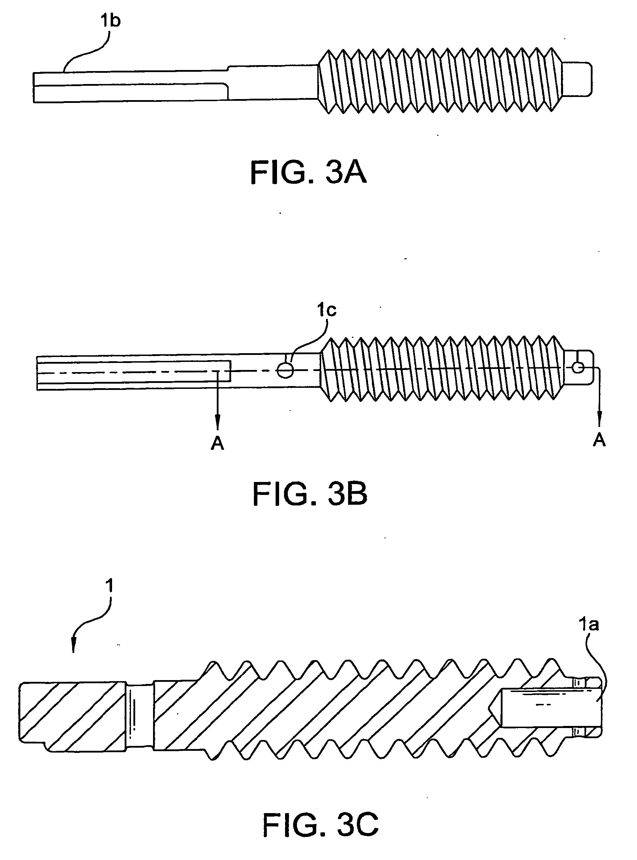

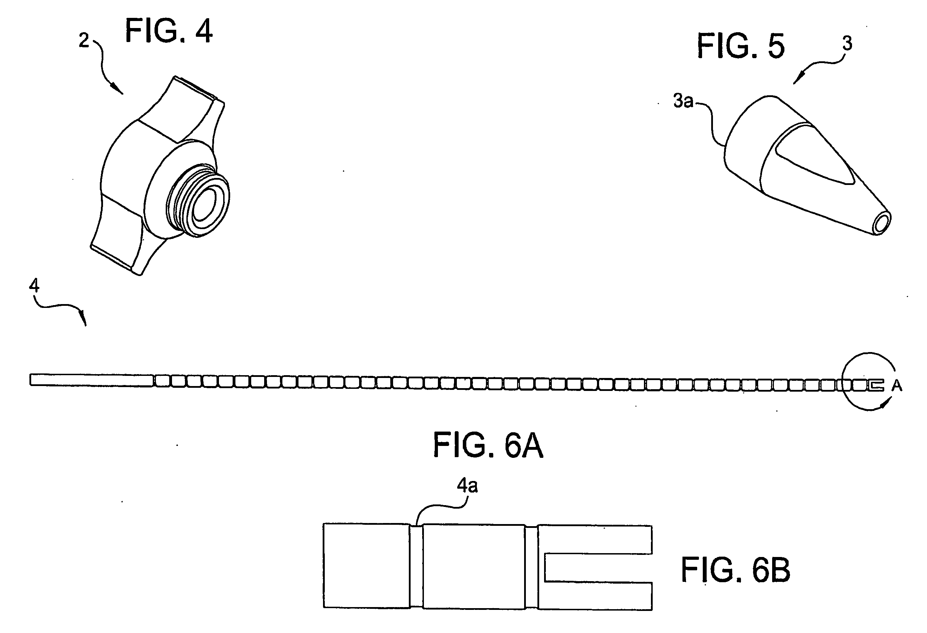

Referring now to the drawings, where like elements are designated by like reference numerals, Figures 1-8 illustrate various components of a flip retrograde cutter 100 of the present invention. The flip retrograde cutter 100 creates a recipient site socket from the inside out, i.e., using a retrograde technique, with minimal incisions of distal cortices and reduced intraarticular bone fragmentation of tunnel rims.

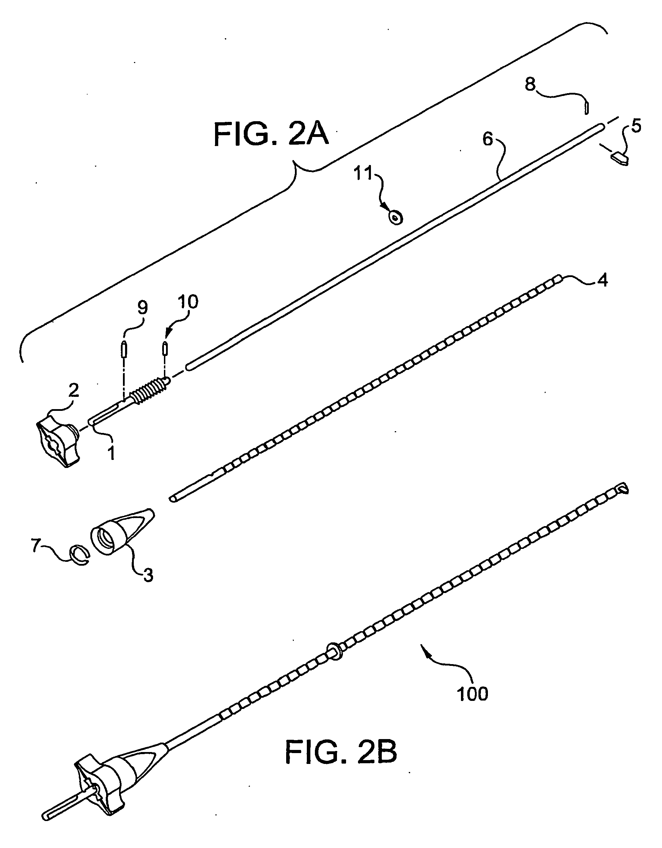

The flip retrograde cutter 100 includes a cannulated elongated body 100c having a distal end 100a and a proximal end 100b, as shown in Figure 1. The body 100c of the flip retrograde cutter 100 includes a shaft 6 and a locking tube 4, with a blade 5 at the distal end 100a of the instrument, as shown in Figures 2A-2B. The following is a list of parts of the flip retrograde cutter 100: driver end 1, nut 2, hub 3, locking tube 4, blade/cutter tip 5, shaft 6, retainer ring 7, cross pin 8, slotted spring pin 9, pin 10 and O-Ring 11. Details of the various parts of the flip retrograde cutter 100 are illustrated in Figures 2-8 and listed below in Table 1.

**Table 1: Apparatus of the present invention**

| Flip Retrograde Cutter | |
|---|---|
| Overall dimensions: | 344,9 mm (13.58 inches) (length) x 3.5 mm (shaft diameter) |
| | 273 mm (10.75 inches) (length from hub to blade) |
| Cutting diameter: | 6-13 mm. Blade rotates freely and locks in straight and 90 degree positions. |

| Driver End | |
|---|---|
| Overall dimensions: | 53 mm (2.1 inches) (length) x 3,96 mm (0.156 inches) (diameter of unthreaded portion) |
| Thread size: | 1/4-20 UNC-2A (6,35 mm) (1/4 inches) diameter with 0,8 threads/mm (20 threads/inch). The threads start 2,5 mm (0.1 inch) from one end. |
| Unthreaded portion: | 28,7 mm (1.13 inches) (length) with a through hole of 1,6 mm (0.063 inches) diameter at 24,3 mm (0.96 inch) from one end. |
| Material: | 18-8 Stainless Steel; clean and electropolish finish |

| Nut | |
|---|---|
| Overall dimensions: | 9,27 mm (0.365 inches) (inner diameter of through hole) x 14,9 mm (0.585 inches) (width). |
| Thread Size: | 1/4-20 UNC-2B |
| Material: | Polylac PA-747 Acrylonitrile butadiene styrene (ABS) |
| Color: | Blue |

| Hub | |
|---|---|
| First Portion: | 15,9 mm (0.625 inches) (outer diameter) x 9,73 mm (0.383 inches) (height) x 2° slope between the diameters at both ends of the first portion. |
| Second Portion: | 24,9 mm (0.979 inches) (height) x 24° slope between the diameters at both ends of the first portion. |
| Material: | Polylac PA-747 Acrylonitrile butadiene styrene (ABS) |
| Color: | Blue |

| Locking Tube | |
|---|---|
| Overall dimensions: | 3,40 mm (0.134 inches) (diameter) x 281 mm (11.05 inches) (length) |
| | Tube has a slot on one end having a width of 1 mm (0.04 inches) and a length of 4,45 mm (0.175 inches) from the end. |
| | Laser etch lines around circumference of the tube. |
| Material: | 17-7 Stainless Steel Condition A; clean and electropolish finish. |

| Blade | |
|---|---|
| Overall dimensions: | 118° (tip angle) x 3,51 mm (0.138 inches) (cutting radius) |
| Effective cutting diameter: | 6-13 mm |
| Material: | 17-4 PH Stainless Steel; clean and electropolish finish |

| Shaft | |
|---|---|
| Overall dimensions: | 2,59 mm (0.102 inches) (diameter) x 295,3 mm (11.625 inches) (length). |
| | One end of the shaft has a through hole 3,8 mm (0.15 inches) from the end and having a diameter of 1 mm (0.04 inches). Another end has a slot with a slot width of 1 mm (0.04 inches) and a length of 4,45 mm (0.175 inches) from the end. The slotted end also has a through hole having a diameter of 1 mm (0.04 inches). |
| Material: | 17-4 PH Stainless Steel; clean and electropolish finish. |
| | |

| Retainer Ring | |
|---|---|
| Overall dimensions: | 12,1 mm (0.475 inches) (outer diameter) x 9,53 mm (0.375 inches) (inner diameter) x 3,18 mm (0.125 inches) (slot gap) x 0,81 mm (0.032 inches) (thickness). |
| Material: | Polylac PA-747 Acrylonitrile Butadiene Styrene (ABS) |
| Color: | Blue |

| Cross Pin | |
|---|---|
| Overall dimensions: | 1,006 mm (0.0396 inches) (diameter) x 2,5 mm (0.1 inches) (length) |
| Material: | 18-8 Stainless Steel |

| Slotted Spring Pin | |
|---|---|
| Overall dimensions: | 1,6 mm (1/16 inches) (diameter) x 9,5 mm (3/8 inches) (length) |
| Material: | 18-8 Stainless Steel |

| Pin | |
|---|---|
| Overall dimensions: | 1 mm x 4 mm |
| Material: | 18-8 Stainless Steel |

| O-Ring | |
|---|---|
| Overall dimensions: | 6,4 mm (0.25 inches) (outer diameter) x 3,2 mm (0.125 inches) (inner diameter) |
| Basic shape: | Round in plan shape |
| Material: | Viton |
| Color: | Black |

The flip retrograde cutter 100 is preferably assembled by first pressing the locking tube 4 into the hub 3 until the locking tube 4 bottoms out. The retainer ring 7 is then inserted into a groove 3a of the hub 3 to form a first sub-assembly.

Next, the shaft 6 is slide into end 1a of the driver end 1 and securely engaged using the pin 10. The first sub-assembly, described above, is slide over the assembled shaft 6 and driver end 1. Next, the blade 5 is slide into slot 6a of the shaft 6 and securely engaged using the cross pin 8, subsequently welding in place the cross pin 8 at both its ends. The nut 2 is then screwed onto the driver end 1. Next, the hub 3 is pulled back while advancing the nut 2 until the retainer ring 7 engages and the slotted spring pin 9 is inserted into a through hole 1c in the driver end 1 to secure the driver end 1 against the nut 2. The O-Ring 11 is slid over the locking tube 4, having laser etches 4a on its circumference, until the O-Ring 11 is about 3-5 inches from the hub 3. The O-Ring 11 may be used to measure depth during retrograde drilling.

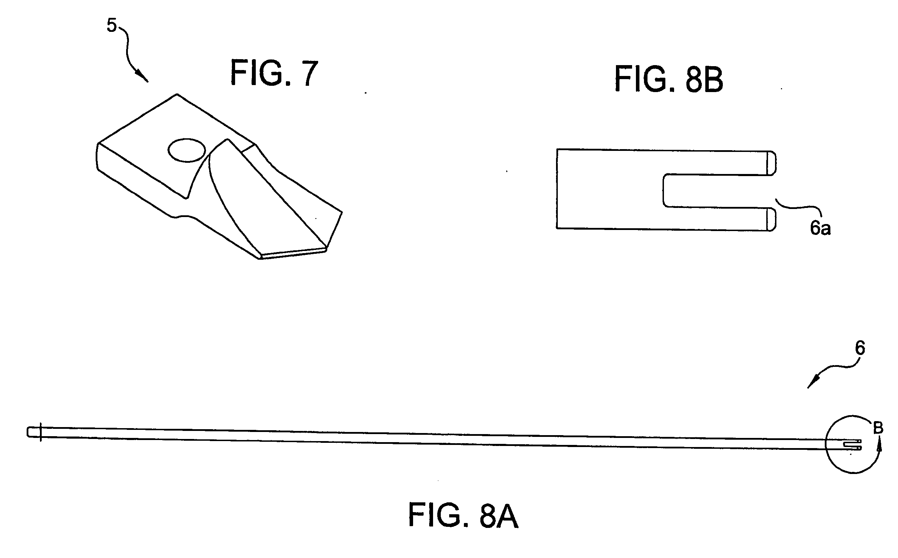

Details of the blade 5 of the flip retrograde cutter 100 are illustrated in Figure 7; however, the invention contemplates other shapes and geometries for the blade 5. The blade 5 is configured to engage the shaft 6 and to articulate between at least first and second positions. Blade 50 engages shaft 11 in a first or "straight" position (FIG. 1A) about parallel to the longitudinal axis of the cutting instrument 100. The blade 50 also engages shaft 6 in a second or "flip" position (FIG. 1B) about 90 degrees relative to the longitudinal axis of the cutting instrument 100.

In use, once the flip retrograde cutter 100 is inserted into a joint, for example, a knee joint, the surgeon rotates the nut 2 to allow the locking tube 4 of the flip retrograde cutter 100 to retract. The blade 5 is then articulated, for example by manipulating it with another instrument, so that it is pivoted into the "flip" position, i.e., into a position other than the "straight" position, about 90 degrees to the longitudinal axis of the instrument. Once the blade 5 is articulated in the desired "flip" position, the blade 5 is preferably locked by tightening the locking tube 4. A drilling operation, for example, a retrodrilling step, may be subsequently carried, as known in the art.

The flip retrograde cutter 100 of the present invention may be employed in a retrograde manner to form a recipient socket (at the location of an osteochondral lesion developed on the head of the tibia, for example, or to accommodate retrograde fixation of a graft within two sockets). Formation of the recipient socket begins by inserting the flip retrograde cutter 100 in the "straight" configuration into the joint space, preferably from the outside in, through a small diameter tunnel (for example, of less than about 4mm). The locking tube 4 of the instrument is then retracted and the blade 5 is articulated into the "flip" configuration. The "flip" position is preferably locked by tightening the locking tube 4 to allow a drilling operation to take place.

The present invention may be used to form various sockets or tunnels for graft fixation or to create sockets in a retrograde manner for replacement osteochondral cores or implants, obviating the need for inserting harvesters into the joint. For example, the flip retrograde cutting instrument 100 of the present invention may be employed for the formation of sockets during an "All-Inside ACL RetroConstruction" ligament repair, by drilling at least a femoral and tibial tunnel or socket using a retrodrill technique employing the flip retrograde cutting instrument 100 of Figures 1-2. A graft (soft tissue graft or BTB graft) may be provided in the vicinity of the sockets and the graft secured within the femoral and tibial tunnels (sockets).

An exemplary method of ACL RetroConstruction of the present invention includes drilling a femoral socket, and drilling a tibial tunnel or socket using a retrodrill technique employing the flip retrograde cutting instrument 100 of Figures 1-2. Next, a graft (soft tissue graft or BTB graft) is provided in the vicinity of the sockets and the graft is secured to a continuous loop/button construct having a button with an oblong configuration and provided with an inside eyelet that allows the passage of the continuous loop, preferably a suture loop. The graft is passed with the button through the femoral tunnel and the button is secured to the femoral cortex once the button exits the femoral socket. Finally, the graft is secured in the tibial tunnel or socket.

Although the above-detailed methods of socket formation using the flip retrograde cutter 100 of the present invention have been described with reference to a specific ACL reconstruction, i.e., a specific "all-inside ACL RetroConstruction" for ligament repair, the invention is not limited thereto and may be used in any repairs and reconstructions that employ a cutting instrument such as flip retrograde cutter 100 of the present invention.

Although the present invention has been described in relation to particular embodiments thereof, many other variations and modifications and other uses will become apparent to those skilled in the art. It is preferred, therefore, that the present invention be limited not by the specific disclosure herein, but only by the appended claims.

## Claims

1. A flip retrograde cutter (100) comprising:
a cannulated elongated body (100c) having a distal end (100a), a proximal end (100b) and a longitudinal axis, the body (100c) comprising
a shaft (6); and
a blade (5) at a distal end of the shaft (6), the blade (5) being securely engaged to the shaft (6) and capable of movement from a first position generally aligned with the longitudinal axis of the body (100c) to a second, flip position which is not aligned with the longitudinal axis; and
further comprising a locking tube (4) over the shaft (6), the locking tube (4) being adapted to securely lock the blade (5) in the first position or the second position; the blade (5), in its second position, being about 90° relative to the longitudinal axis of the shaft (6), the blade (5) thereby being adapted to retrograde cut as the flip retrograde cutter (100) is pulled proximally;
**characterized in that** the locking tube (4) has a slot at its distal end adapted to securely lock the blade (5) in the first position or the second position.

2. The flip retrograde cutter (100) of ctaim 1 further comprising:
a hub (3) attached to a proximal end of the locking tube (4);
a driver end (1) having screw threads in a portion near a distal end of the driver end (1) and a cannula at the distal end (1a) of the driver end (1), a proximal end of the shaft (6) being inserted into the cannula; and
a nut (2) to securely engage the screw threads of the driver end (1) and the hub (3).

3. The flip retrograde cutter (100) of claim 1 or claim 2, further comprising an O-Ring (11), the O-Ring (11) being placed over the locking tube (4), wherein the O-Ring (11) is used to measure depth during retrograde drilling.

4. The flip retrograde cutter (100) of claim 1, wherein the locking tube (4) is retracted by rotating a nut (2).

5. The flip retrograde cutter (100) of claim 2 or claims 2 and 3, wherein the locking tube (4) is retracted by rotating the nut (2).

6. The flip retrograde cutter (100) of any one of the preceding claims, wherein the locking tube (4) has laser etch lines (4a) around its circumference.

7. The flip retrograde cutter (100) of any one of the preceding claims, wherein the blade (5) has a cutting diameter of about 6 mm to about 13 mm.

8. The flip retrograde cutter (100) of any one of the preceding claims, wherein the shaft (6) is made of stainless steel.

9. The flip retrograde cutter (100) of any one of the preceding claims, wherein the shaft (6) has a diameter of about 3.5 mm.

## Patentansprüche

1. Ausklappbares rückseitiges Schneidinstrument (100), das aufweist:
einen kannulierten länglichen Körper (100c) mit einem distalen Ende (100a), einem proximalen Ende (100b) und einer Längsachse, wobei der Körper (100c) aufweist:
einen Schaft (6); und
eine Klinge (5) an einem distalen Ende des Schaftes (6), wobei die Klinge (5) sicher mit dem Schaft (6) in Eingriff gebracht wird und zu einer Bewegung aus einer ersten Position, die im Allgemeinen mit der Längsachse des Körpers (100c) ausgerichtet ist, in eine zweite ausklappbare Position in der Lage ist, die nicht mit der Längsachse ausgerichtet ist; und
das außerdem ein Verriegelungsrohr (4) über dem Schaft (6) aufweist, wobei das Verriegelungsrohr (4) ausgebildet ist, um die Klinge (5) sicher in der ersten Position oder der zweiten Position zu verriegeln,
wobei die Klinge (5) in seiner zweiten Position etwa 90° relativ zur Längsachse des Schaftes (6) ist, wodurch die Klinge (5) ausgebildet ist, um rückseitig zu schneiden, während das ausklappbare rückseitige Schneidinstrument (100) proximal herausgezogen ist;
**dadurch gekennzeichnet, dass** das Verriegelungsrohr (4) einen Schlitz an seinem distalen Ende aufweist, der ausgebildet ist, um die Klinge (5) in der ersten Position oder der zweiten Position sicher zu verriegeln.

2. Ausklappbares rückseitiges Schneidinstrument (100) nach Anspruch 1, das außerdem aufweist:
eine Nabe (3), die an einem proximalen Ende des Verriegelungsrohres (4) befestigt ist;
ein Steuerende (1), das Schraubengewindegänge in einem Abschnitt in der Nähe eines distalen Endes des Steuerendes (1) und eine Kanüle am distalen Ende (1a) des Steuerendes (1) aufweist, wobei ein proximales Ende des Schaftes (6) in die Kanüle eingesetzt wird; und
eine Mutter (2), um die Schraubengewindegänge des Steuerendes (1) und der Nabe (3) sicher in Eingriff zu bringen.

3. Ausklappbares rückseitiges Schneidinstrument (100) nach Anspruch 1 oder Anspruch 2, das außerdem einen O-Ring (11) aufweist, wobei der O-Ring (11) über dem Verriegelungsrohr (4) angeordnet wird, wobei der O-Ring (11) verwendet wird, um die Tiefe während des rückseitigen Bohrens zu messen.

4. Ausklappbares rückseitiges Schneidinstrument (100) nach Anspruch 1, bei dem das Verriegelungsrohr (4) durch Drehen einer Mutter (2) zurückgezogen wird.

5. Ausklappbares rückseitiges Schneidinstrument (100) nach Anspruch 2 oder den Ansprüchen 2 und 3, wobei das Verriegelungsrohr (4) durch Drehen der Mutter (2) zurückgezogen wird.

6. Ausklappbares rückseitiges Schneidinstrument (100) nach einem der vorhergehenden Ansprüche, bei dem das Verriegelungsrohr (4) Laserätzlinien (4a) um seinen Umfang aufweist.

7. Ausklappbares rückseitiges Schneidinstrument (100) nach einem der vorhergehenden Ansprüche, bei dem die Klinge (5) einen Schneiddurchmesser von etwa 6 mm bis etwa 13 mm aufweist.

8. Ausklappbares rückseitiges Schneidinstrument (100) nach einem der vorhergehenden Ansprüche, bei dem der Schaft (6) aus nichtrostendem Stahl besteht.

9. Ausklappbares rückseitiges Schneidinstrument (100) nach einem der vorhergehenden Ansprüche, bei dem der Schaft (6) einen Durchmesser von etwa 3,5 mm aufweist.

## Revendications

1. Instrument de coupe rétrograde basculant (100), comprenant :
un corps allongé en forme de canule (100c), comportant une extrémité distale (100a), une extrémité proximale (100b) et un axe longitudinal, le corps (100c) comprenant :
une tige (6) ; et
une lame (5) au niveau d'une extrémité distale de la tige (6), la lame (5) étant engagée fermement dans la tige (6) et pouvant se déplacer d'une première position, généralement alignée avec l'axe longitudinal du corps (100c), vers une deuxième position à basculement, non alignée avec l'axe longitudinal ; et
comprenant en outre un tube de verrouillage (4) au-dessus de la tige (6), le tube de verrouillage (4) étant adapté pour verrouiller fermement la lame (5) dans la première position ou dans la deuxième position ;
la lame (5) formant, dans sa deuxième position, un angle d'environ 90° par rapport à l'axe longitudinal de la tige (6), la lame (5) étant ainsi adaptée pour effectuer une coupe rétrograde lorsque l'instrument de coupe rétrograde basculant (100) est tiré dans une direction proximale ;
**caractérisé en ce que** le tube de verrouillage (4) comporte une fente au niveau de son extrémité distale, adaptée pour verrouiller fermement la lame (5) dans la première position ou dans la deuxième position.

2. Instrument de coupe rétrograde basculant (100) selon la revendication 1, comprenant en outre :
un moyeu (3), fixé sur une extrémité proximale du tube de verrouillage (4) ;
une extrémité d'entraînement (1) comportant des filets de vis dans une partie proche d'une extrémité distale de l'extrémité d'entraînement (1), et une canule au niveau de l'extrémité distale (1a) de l'extrémité d'entraînement (1), une extrémité proximale de la tige (6) étant insérée dans la canule ; et
un écrou (2), en vue d'un engagement ferme des filets de vis de l'extrémité d'entraînement (1) et du moyeu (3).

3. Instrument de coupe rétrograde basculant (100) selon les revendications 1 ou 2, comprenant en outre un joint torique d'étanchéité (11), le joint torique d'étanchéité (11) étant placé au-dessus du tube de verrouillage (4), le joint torique d'étanchéité (11) étant utilisé pour mesurer la profondeur au cours du perçage rétrograde.

4. Instrument de coupe rétrograde basculant (100) selon la revendication 1, dans lequel le tube de verrouillage (4) est rétracté par rotation d'un écrou (2).

5. Instrument de coupe rétrograde basculant (100) selon la revendication 2 ou les revendications 2 et 3, dans lequel le tube de verrouillage (4) est rétracté par rotation de l'écrou (2).

6. Instrument de coupe rétrograde basculant (100) selon l'une quelconque des revendications précédentes, dans lequel le tube de verrouillage (4) comporte des lignes de gravure au laser (4a) autour de sa circonférence.

7. Instrument de coupe rétrograde basculant (100) selon l'une quelconque des revendications précédentes, dans lequel la lame (5) a un diamètre de coupe compris entre environ 6 mm et environ 13 mm.

8. Instrument de coupe rétrograde basculant (100) selon l'une quelconque des revendications précédentes, dans lequel la tige (6) est composé d'acier inoxydable.

9. Instrument de coupe rétrograde basculant (100) selon l'une quelconque des revendications précédentes, dans lequel la tige (6) a un diamètre d'environ 3,5 mm.
